# EUROPÄISCHE PATENTSCHRIFT

(11) **EP 1 161 420 B1**
(45) Veröffentlichungstag und Bekanntmachung des Hinweises auf die Patenterteilung: **11.05.2005**
(21) Anmeldenummer: 99968665.2
(22) Anmeldetag: 23.08.1999
(51) Int. Cl.: C07D 231/16, A01N 43/56

(54) **PYRAZOL-CARBOXANILIDE FUNGIZIDE**
FUNGICIDAL PYRAZOLE CARBOXANILIDES
CARBOXANILIDES DE PYRAZOLE FONGICIDES

(30) Priorität: 04.09.1998 DE 19840322
(43) Veröffentlichungstag der Anmeldung: 12.12.2001
(73) Patentinhaber: Bayer CropScience AG, 40789 Monheim (DE)
(72) Erfinder: ELBE, Hans-Ludwig, D-42329 Wuppertal (DE); MAULER-MACHNIK, Astrid, D-42799 Leichlingen (DE); STENZEL, Klaus, D-40595 Düsseldorf (DE); KUCK, Karl-Heinz, D-40764 Langenfeld (DE); JAETSCH, Thomas, D-50668 Köln (DE); KUGLER, Martin, D-42799 Leichlingen (DE)
(86) Internationale Anmeldenummer: PCT/EP1999/006149
(87) Internationale Veröffentlichungsnummer: WO 2000/014071

(56) Entgegenhaltungen:
- EP-A- 0 589 301
- EP-A- 0 776 889
- WO-A-93/11117
- WO-A-97/08148
- WO-A-98/03500
- WO-A-99/09013
- DATABASE CHEMABS [Online] CHEMICAL ABSTRACTS SERVICE, COLUMBUS, OHIO, US; YOSHIKAWA, YUKIHIRO ET AL: "Preparation of pyrazolecarboxyanilide derivatives as agrochemical fungicides" retrieved from STN Database accession no. 127:5086 XP002135650 -& JP 09 132567 A (MITSUI TOATSU CHEMICALS, INC., JAPAN) 20. Mai 1997 (1997-05-20)

## Beschreibung

Die vorliegende Erfindung betrifft neue Pyrazol-carboxanilide, ein Verfahren zu deren Herstellung und deren Verwendung zur Bekämpfung von unerwünschten Mikroorganismen.

Es ist bereits bekannt geworden, daß zahlreiche Carboxanilide fungizide Eigenschaften besitzen (vergleiche WO 93/11 117, EP-A 0 545 099 und EP-A 0 589 301). So lassen sich 1,3-Dimethyl-5-fluorpyrazol-4-carbonsäure-(2-cyclohexyl)-anilid, 1,3-Dimethyl-pyrazol-4-carbonsäure-(2-phenyl)-anilid und 1,3-Dimethyl-pyrazol-4-carbonsäure-[2-(2-fluor-phenyl)]-anilid zur Bekämpfung von Pilzen einsetzen. Die Wirksamkeit dieser Stoffe ist gut, läßt aber bei niedrigen Aufwandmengen in manchen Fällen zu wünschen übrig.

Es wurden nun neue Pyrazol-carboxanilide der Formel 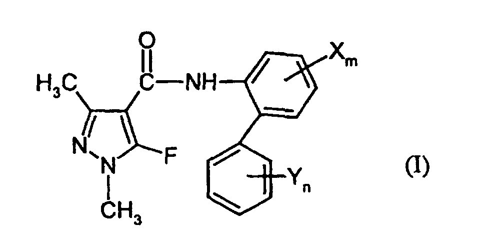 in welcher
a)
   - m: für die Zahl 0 steht,
   - n: für die Zahl 1 steht und
   - Y: für 2-Chlor, 2-Fluor, 4-Brom, 2-Methyl, 2-Trifluormethyl, 3-Trifluormethyl, für Nitro, Cyano, Hydroxy, Carboxyl, Halogenalkoxy mit 1 bis 6 Kohlenstoffatomen und 1 bis 5 Halogenatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
   oder
b)
   - m: für die Zahl 0 steht,
   - n: für die Zahlen 2 oder 3 steht und
   - Y: für Halogen, Nitro, Cyano, Hydroxy, Carboxyl, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
   oder
c)
   - m: für die Zahl 1 steht,
   - X: für Chlor, Brom, Nitro, Cyano, Hydroxy, Carboxyl, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
   - n: für ganze Zahlen von 0 bis 3 steht und
   - Y: für Halogen, Nitro, Cyano, Hydroxy, Carboxyl, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
gefunden.

Weiterhin wurde gefunden, daß man Pyrazol-carboxanilide der Formel (I) erhält, indem man Säurehalogenide der Formel 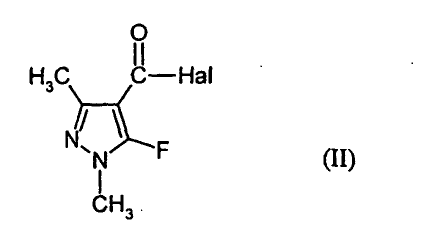 in welcher
Hal für Halogen steht,
mit Anilin-Derivaten der Formel 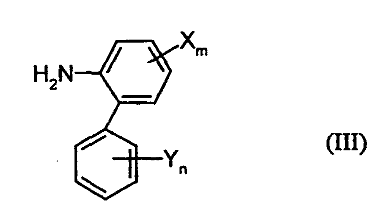 in welcher
X, Y, m und n die oben angegebenen Bedeutungen haben,
gegebenenfalls in Gegenwart eines Säurebindemittels und gegebenenfalls in Gegenwart eines Verdünnungsmittels umsetzt.

Schließlich wurde gefunden, daß die neuen Pyrazol-carboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Pyrazol-carboxanilide der Formel (I) eine wesentlich bessere fungizide Wirksamkeit als 1,3-Dimethyl-5-fluorpyrazol-4-carbonsäure-(2-cyclohexyl)-anilid, 1,3-Dimethyl-pyrazol-4-carbonsäure-(2-phenyl)-anilid und 1,3-Dimethyl-pyrazol-4-carbonsäure-[2-(2-fluor-phenyl)]-anilid, welches die konstitutionell ähnlichsten, vorbekannten Wirkstoffe gleicher Wirkungsrichtung sind.

Die erfindungsgemäßen Pyrazol-carboxanilide sind durch die Formel (I) allgemein definiert.

Bevorzugt sind Verbindungen der Formel (I), in denen
a)
   - m: für die Zahl 0 steht,
   - n: für die Zahl 1 steht und
   - Y: für 2-Chlor, 2-Fluor, 4-Brom, 2-Methyl, 2-Trifluormethyl, 3-Trifluormethyl,
   für Nitro, Cyano, Hydroxy, Carboxyl, Halogenalkoxy mit 1 oder 2 Kohlenstoffatomen und 1 bis 5 Fluor-, Chlor- und/oder Bromatomen, Halogenalkylthio mit 1 oder 2 Kohlenstoffatomen und 1 bis 5 Fluor-, Chlor und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
   oder
b)
   - m: für die Zahl 0 steht,
   - n: für die Zahlen 2 oder 3 steht und
   - Y: für Fluor, Chlor, Brom, Nitro, Cyano, Hydroxy, Carboxyl, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor- und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
   oder
c)
   - m: für die Zahl 1 steht,
   - X: für Chlor, Brom, Nitro, Cyano, Hydroxy, Carboxyl, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
   - n: für ganze Zahlen von 0 bis 3 steht und
   - Y: für Fluor, Chlor, Brom, Nitro, Cyano, Hydroxy, Carboxyl, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor- und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht.

Besonders bevorzugt sind Verbindungen der Formel (I), in denen
a)
   - m: für die Zahl 0 steht,
   - n: für die Zahl 1 steht und
   - Y: für 2-Chlor, 2-Fluor, 4-Brom, 2-Methyl, 2-Trifluormethyl, 3-Trifluormethyl,
   für Nitro, Cyano, Hydroxy, Carboxyl, Difluormethoxy, Trifluormethoxy, Trifluormethylthio, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl steht,
   oder
b)
   - m: für die Zahl 0 steht,
   - n: für die Zahlen 2 oder 3 steht und
   - Y: für Fluor, Chlor, Brom, Nitro, Cyano, Hydroxy, Carboxyl, Methyl, Ethyl, n-Propyl, Isopropyl, n-Butyl, sek.-Butyl, i-Butyl, tert.-Butyl, Trichlormethyl, Trifluormethyl, Difluormethyl, Difluorchlormethyl, Methoxy, Ethoxy Methylthio, Difluormethoxy, Trifluormethoxy, Trifluormethylthio, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl steht,
   oder
c)
   - m: für die Zahl 1 steht,
   - X: für Chlor, Brom, Nitro, Cyano, Hydroxy, Carboxyl, Methyl, Ethyl, n-Propyl, Isopropyl, n-Butyl, sek.-Butyl, i-Butyl, tert.-Butyl, Trichlormethyl, Trifluormethyl, Difluormethyl, Difluorchlormethyl, Methoxy, Ethoxy, Methylthio, Difluormethoxy, Trifluormethoxy, Trifluormethylthio, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl steht,
   - n: für ganze Zahlen von 0 bis 3 steht und
   - Y: für Fluor, Chlor, Brom, Nitro, Cyano, Hydroxy, Carboxyl, Methyl, Ethyl, n-Propyl, Isopropyl, n-Butyl, sek.-Butyl, i-Butyl, tert.-Butyl, Trichlormethyl, Trifluormethyl, Difluormethyl, Difluorchlormethyl, Methoxy, Ethoxy Methylthio, Difluormethoxy, Trifluormethoxy, Trifluormethylthio, Difluorchlormethylthio, Allyloxy, Propargyloxy, Cyclopropyl, Cyclopentyl, Cyclohexyl, Cycloheptyl, Methoxycarbonyl, Ethoxycarbonyl, Methoximinomethyl, Ethoximinomethyl, Methoximinoethyl oder Ethoximinoethyl steht.

Die genannten Definitionen können untereinander in beliebiger Weise kombiniert werden. Außerdem können auch einzelne Definitionen entfallen.

Verwendet man 1,3-Dimethyl-5-fluorpyrazol-4-carbonsäurechlorid und 2-(4-Bromphenyl)-anilin als Ausgangsstoffe, so kann der Verlauf des erfindungsgemäßen Verfahrens durch das folgende Formelschema veranschaulicht werden.

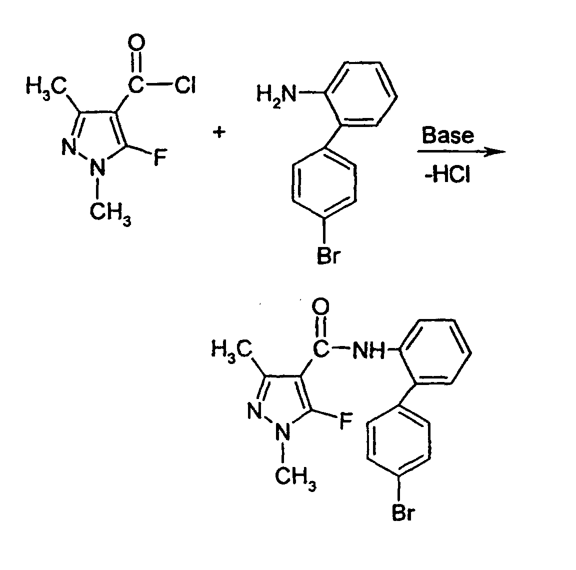

Die bei der Durchführung des erfindungsgemäßen Verfahrens als Ausgangsstoffe benötigten Säurehalogenide sind durch die Formel (II) allgemein definiert. In dieser Formel steht Hal vorzugsweise für Fluor, Chlor oder Brom.

Die Säurehalogenide der Formel (II) sind bekannt oder lassen sich nach bekannten Verfahren herstellen (vgl. WO 93/11 117 und EP-A 0 776 889).

Die bei der Durchführung des erfindungsgemäßen Verfahrens als Reaktionskomponenten benötigten Anilin-Derivate sind durch die Formel (III) allgemein definiert. In dieser Formel haben X, Y, m und n vorzugsweise diejenigen Bedeutungen, die bereits im Zusammenhang mit der Beschreibung der erfindungsgemäßen Stoffe der Formel (I) vorzugsweise für diese Reste bzw. diese Indices genannt wurden.

Die Anilin-Derivate der Formel (III) sind bekannt oder lassen sich nach bekannten Methoden herstellen (vgl. EP-A 0 545 099 und EP-A 0 589 301).

Als Säurebindemittel kommen bei der Durchführung des erfindungsgemäßen Verfahrens alle für derartige Reaktionen üblichen anorganischen und organischen Basen in Betracht. Vorzugsweise verwendbar sind Erdalkali- oder Alkalimetallhydroxide, wie Natriumhydroxid, Calciumhydroxid, Kaliumhydroxid, oder auch Ammoniumhydroxid, Alkalimetallcarbonate, wie Natriumcarbonat, Kaliumcarbonat, Kaliumhydrogencarbonat, Natriumhydrogencarbonat, Alkali- oder Erdalkalimetallacetate wie Natriumacetat, Kaliumacetat, Calciumacetat, sowie tertiäre Amine, wie Trimethylamin, Triethylamin, Tributylamin, N,N-Dimethylanilin, Pyridin, N-Methylpiperidin, N,N-Dimethylaminopyridin, Diazabicyclooctan (DABCO), Diazabicyclononen (DBN) oder Diazabicycloundecen (DBU). Es ist jedoch auch möglich, ohne zusätzliches Säurebindemittel zu arbeiten, oder die Aminkomponente in einem Überschuß einzusetzen, so daß sie gleichzeitig als Säurebindemittel fungiert.

Als Verdünnungsmittel kommen bei der Durchführung des erfindungsgemäßen Verfahrens alle üblichen inerten, organischen Solventien in Frage. Vorzugsweise verwendbar sind gegebenenfalls halogenierte aliphatische, alicyclische oder aromatische Kohlenwasserstoffe, wie Petrolether, Hexan, Heptan, Cyclohexan, Methylcyclohexan, Benzol, Toluol, Xylol oder Decalin; Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid oder Sulfone, wie Sulfolan.

Die Reaktionstemperaturen können bei der Durchführung des erfindungsgemäßen Verfahrens in einem größeren Bereich variiert werden. Im allgemeinen arbeitet man bei Temperaturen zwischen 0°C und 140°C, vorzugsweise zwischen 10°C und 120°C.

Bei der Durchführung des erfindungsgemäßen Verfahrens arbeitet man im allgemeinen unter Atmosphärendruck. Es ist aber auch möglich, jeweils unter erhöhtem oder vermindertem Druck zu arbeiten.

Bei der Durchführung des erfindungsgemäßen Verfahrens setzt man auf 1 Mol an Säurehalogenid der Formel (II) im allgemeinen 1 Mol oder auch einen Überschuß an Anilin-Derivat der Formel (III) sowie 1 bis 3 Mol an Säurebindemittel ein. Es ist jedoch auch möglich, die Reaktionskomponenten in anderen Verhältnissen einzusetzen. Die Aufarbeitung erfolgt nach üblichen Methoden. Im allgemeinen verfährt man in der Weise, daß man das Reaktionsgemisch mit Wasser versetzt, die organische Phase abtrennt und nach dem Trocknen unter vermindertem Druck einengt. Der verbleibende Rückstand kann gegebenenfalls nach üblichen Methoden, wie Chromatographie oder Umkristallisation, von eventuell noch vorhandenen Verunreinigungen befreit werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau einsetzen, wie beispielsweise gegen Venturia-, Botrytis-, Sclerobinia-, Rhizoctonia-, Uncinula-, Sphaerotheca-, Podosphaera-, Altemaria- und Colletotriclum-Arten. Mit gutem Erfolg werden auch Reiskrankeiten, wie Pyricularia- und Pellicularia-Arten, bekämpft.

Die erfindungsgemäßen Wirkstoffe eignen sich auch zur Steigerung des Ernteertrages. Sie sind außerdem mindertoxisch und weisen eine gute Pflanzenverträglichkeit auf

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitem oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen (PCNB), Quinoxyfen,
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-b-propyl-1H-1,2,4-triazol-1-ethanol,
α-(2,4-Dichlorphenyl)-β-methoxy-a-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-a-(Methoxyimino)-N-methyl-2-phenoxy-phenylacetamid,
{2-Methyl-1-[[[1-(4-methylphenyl)-ethyl]-amino]-carbonyl]-propyl}-carbaminsäure-1-isopropylester,
1-(2,4-Dichlorphenyl)-2-(1H-1,2,4-triazol-1-yl)-ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-[2-[(2,4-Dichlorphenyl)-methoxy]-phenyl]-ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)-ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)-phenyl]-methyl]-benzamid,
2-(2,3,3-Triiod-2-propenyl)-2H-tetrazol,
2-[(1-Methylethyl)-sulfonyl]-5-(trichlormethyl)-1,3,4-thiadiazol,
2-[[6-Deoxy-4-O-(4-O-methyl-β-D-glycopyranosyl)-a-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)-oxy]-methyl]-benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)-oxy]-2,5-thiophendicarboxylat, cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol-Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydre-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis-(2-propinyloxy)-phenyl]-N'-methoxy-methanimidamid,
N-Formyl-N-hydroxy-DL-alanin-Natriumsalz,
O,O-Diethyl-[2-(dipropylamino)-2-oxoethyl]-ethylphosphoramidothioat,
O-Methyl-S-phenyl-phenylpropylphosphoramidothioate,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalarn, Kupfersulfat und andere Kupfer-Zubereitungen.

### Insektizide / Akarizide / Nematizide:

Abamectin, Acephat, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
Bacillus popilliae, Bacillus sphaericus, Bacillus subtilis, Bacillus thuringiensis, Baculoviren; Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, BPMC, Bromophos A, Bufencarb, Buprofezin, Butathiofos, Butocarboxim, Butylpyridaben,
Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaphorthrin, Cis-Resmethrin, Cispermethrin, Clocythrin, Cloethocarb, Clofentezine, Cyanophos, Cycloprene, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlorvos, Diflubenzuron, Dimethoat, Dimethylvinphos, Diofenolan, Disulfoton, Docusat-sodium, Dofenapyn,
Elfusilanate, Emamectin, Empenthrin, Endosulfan, Entomopfthora spp., Esfenvalerate, Ethiofencarb, Ethion, Ethoprophos, Etofenprox, Etoxazole, Etrimphos,
Fenamiphos, Fenazaquin, Fenbutatinoxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazinam, Fluazuron, Flubrocythrinate, Flucycloxuron, Flucythrinate, Flufenoxuron, Flutenzine, Fluvalinate, Fonophos, Fosmethilan, Fosthiazate, Fubfenprox, Furathiocarb,
Granuloseviren,
Halofenozide, HCH, Heptenophos, Hexaflumuron, Hexythiazox, Hydroprene,
Imidacloprid, Isazophos, Isofenphos, Isoxathion, Ivermectin,
Kempolyederviren,
Lamda-cyhalothrin, Lufenuron,
Malathion, Mecarbam, Metaldehyd, Methamidophos, Metharhizium anisopliae, Metharhizium flavoviride, Methidathion, Methiocarb, Methomyl, Methoxyfenozide, Metolcarb, Metoxadiazone, Meviriphos, Milbemectin, Monocrotophos,
Naled, Nitenpyram, Nithiazine, Novaluron,
Omethoat, Oxamyl, Oxydemethon M,
Paecilomyces fumosoroseus, Parathion A, Parathion M, Permethrin, Phenthoat, Phorat, Phosalon, Phosmet, Phosphamidon, Phoxim, Pirimicarb, Pirimiphos A, Pirimiphos M, Profenophos, Promecarb, Propoxur, Prothiophos, Prothoat, Pymetrozine, Pyraclofos, Pyresmethrin, Pyrethrum, Pyridaben, Pyridathion, Pyrimidifen, Pyriproxifen,
Quinalphos,
Ribavirin,
Salithion, Sebufos, Silafluofen, Spinosad, Sulfotep, Sulprofos,
Tau-fluvalinate, Tebufenozide, Tebufenpyrad, Tebupirimiphos, Teflubenzuron, Tefluthrin, Temephos, Temivinphos, Terbufos, Tetrachlorvinphos, Thetacypennethrin, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
Vamidothion, Vaniliprole, Verticillium lecaaii
YI 5302
Zeta-Cypermethrin, Zolaprofos

(1R-cis)-[5-(Phenylmethyl)-3-furanyl]-methyl-3-[(dihydro-2-oxo-3(2H)-furanyliden)-methyl]-2,2-dimethylcyclopropancarboxylat,
(3-Phenoxyphenyl)-methyl-2,2,3,3-tetramethylcyclopropanecarboxylat,
1-[(2-Chlor-5-thiazolyl)methyl]tetrahydro-3,5-dimethyl-N-nitro-1,3,5-triazin-2(1H)-imin,
2-(2-Chlor-6-fluorphenyl)-4-[4-(1,1-diemthylethyl)phenyl]-4,5-dihydro-oxazol,
2-(Acetlyoxy)-3-docecyl-1,4-naphthalinidion,
2-Chlor-N-[[[4-(1-phenylethoxy)-phenyl]-amino]-carbonyl]-benzamid,
2-Chlor-N-[[[4-(2,2-dichlor-1,1-difluorethoxy)-phenyl]-amino]-carbonyl]-benzamid,
3-Methylphenyl-propylcarbamat,
4-[4-(4-Ethoxyphenyl)-4-methylpentyl]-1-fluor-2-phenoxy-benzol,
4-Chlor-2-(1,1-dimethylethyl)-5-[[2-(2,6-dimethyl-4-phenoxyphenoxy)ethyl]thio]-3 (2H)-pyridazinon,
4-Chlor-2-(2-chlor-2-methylpropyl)-5-[(6-iod-3-pyridinyl)methoxy]-3(2H)-pyridazinon,
4-Chlor-5[(6-chlor-3-pyridinyl)methoxy]-2-(3,4-dichlorphenyl)-3(2H)-pyridazinon,
Bacillus thuringiensis strain EG-2348,
Benzoesäure (2-benzoyl-1-(1,1-dimethylethyl)-hydrazid,
Butan 2,2-dimethyl-3-(2,4-dichlorphenyl)-2-oxo-1-oxaspiro[4.5]dec-3-en-4-yl-ester,
[3-[(6-Chlor-3-pyridinyl)methyl]-2-thiazolidinyliden]-cyanamid,
Dihydro-2-(nitromethylen)-2H-1,3-thiazine-3(4H)-carboxaldehyd,
Ethyl-[2-[[1,6-dihydro-6-oxo-1-(phenylmethyl)-4-pyridazinyl]oxy]ethyl]-carbamat,
N-(3,4,4-Trifluor-1-oxo-3-butenyl)-glycin,
N-(4-Chlorphenyl)-3-[4-Difluormethoxy)phenyl]-4,5-dihydro-4-phenyl-1H-pyrazol-1-carboxamid,
N-[(2-Chlor-5-thiazolyl)methyl]-N'-methyl-N"-nitro-guanidin,
N-Methyl-N'-(1-methyl-2-propenyl)-1,2-hydrazindicarbothioamid,
N-Methyl-N'-2-propenyl-1,2-hydrazindicarbothioamid,
O,O-Diethyl-[2-(dipropylamino)-2-oxoethyl]-ethylphosphoramidothioat.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10 000 g/ha, vorzugsweise zwischen 10 und 1 000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10 000 g/ha, vorzugsweise zwischen 1 und 5 000 g/ha.

Die zum Schutz technischer Materialien verwendeten Mittel enthalten die Wirkstoffe im allgemeinen in einer Menge von 1 bis 95 %, bevorzugt von 10 bis 75 %.

Die Anwendungskonzentrationen der erfindungsgemäßen Wirkstoffe richten sich nach der Art und dem Vorkommen der zu bekämpfenden Mikroorganismen sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-% bezogen auf das zu schützende Material.

Die Wirksamkeit und das Wirkungsspektrum der erfindungsgemäß im Materialschutz zu verwendenden Wirkstoffe bzw. der daraus herstellbaren Mittel, Konzentrate oder ganz allgemein Formulierungen kann erhöht werden, wenn gegebenenfalls weitere antimikrobiell wirksame Verbindungen, Fungizide, Bakterizide, Herbizide, Insektizide oder andere Wirkstoffe zur Vergrößerung des Wirkungsspektrums oder Erzielung besonderer Effekte wie z.B. dem zusätzlichen Schutz vor Insekten zugesetzt werden. Diese Mischungen können ein breiteres Wirkungsspektrum besitzen als die erfindungsgemäßen Verbindungen.

Die Herstellung und die Verwendung der erfindungsgemäßen Wirkstoffe geht aus den folgenden Beispielen hervor.

### Herstellungsbeispiele

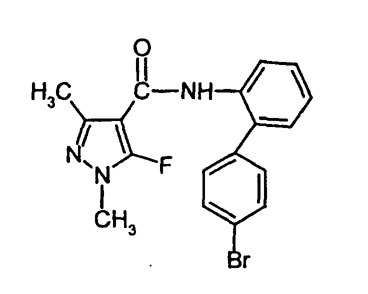

Eine Lösung von 2,5 g (0,01 Mol) 2-(4-Brom-phenyl)-anilin in 30 ml Toluol wird bei Raumtemperatur mit 1,0 g (0,01 Mol) Triethylamin versetzt. In dieses Gemisch werden bei Raumtemperatur unter Rühren 1,8 g (0,01 Mol) 1,3-Dimethyl-5-fluorpyrazol-4-carbonsäurechlorid gegeben. Nach beendeter Zugabe wird das Reaktionsgemisch zwei Stunden bei Raumtemperatur nachgerührt und dann auf Wasser gegossen. Das Gemisch wird mehrfach mit Chloroform extrahiert. Die vereinigten organischen Phasen werden über Natriumsulfat getrocknet, filtriert und unter vermindertem Druck eingeengt. Der verbleibende Rückstand wird mit Diisopropylether verrührt. Das dabei anfallende kristalline Produkt wird abgesaugt und getrocknet. Man erhält auf diese Weise 1,6 g (41,2 % der Theorie) an 1,3-Dimethyl-5-fluor-pyrazol-4-carbonsäure-[2-(4-bromphenyl)]-anilid in Form einer Festsubstanz vom Schmelzpunkt 127°C.

In der zuvor beschriebenen Weise werden auch die in der folgenden Tabelle aufgeführten Pyrazol-carboxanilide der Formel (I) hergestellt.

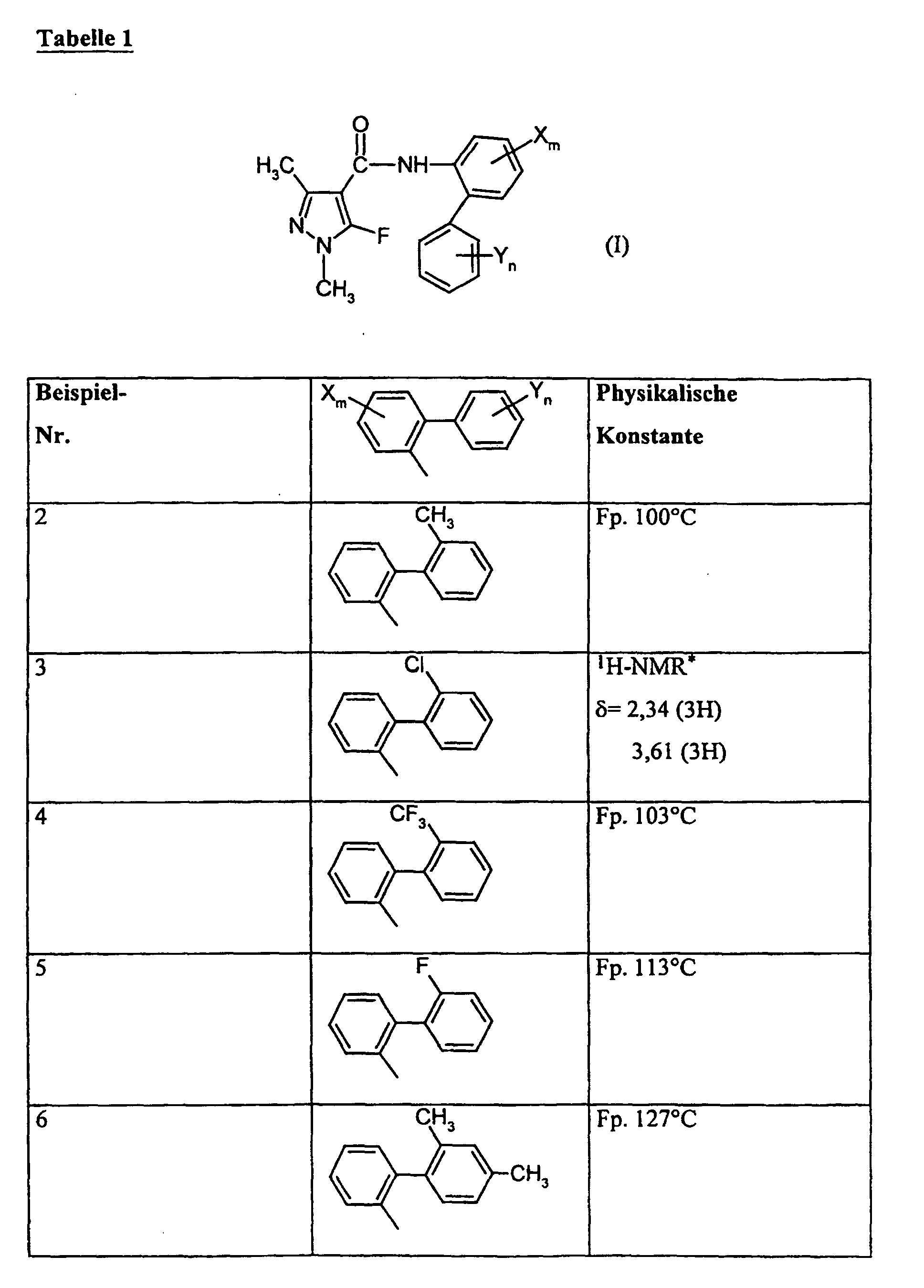

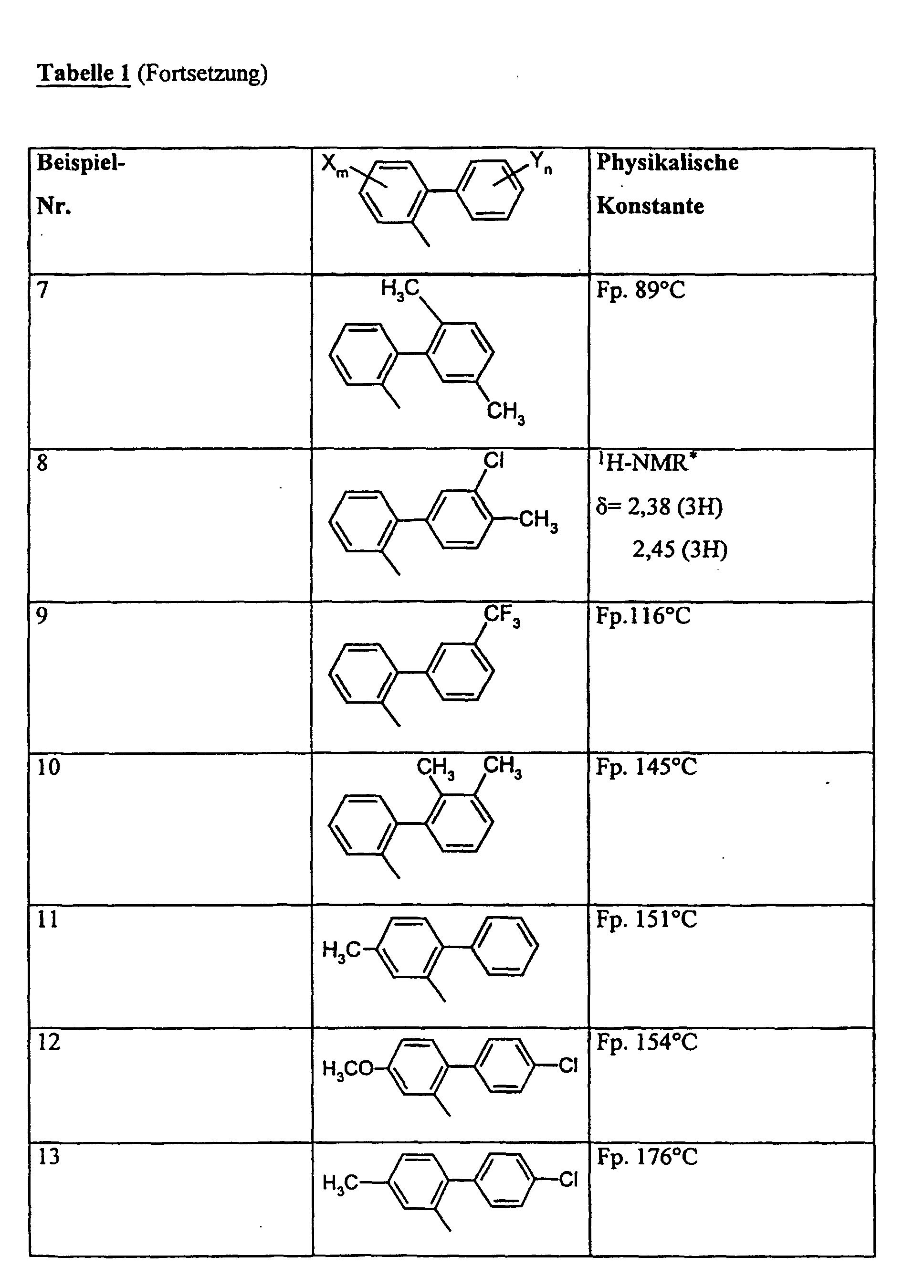

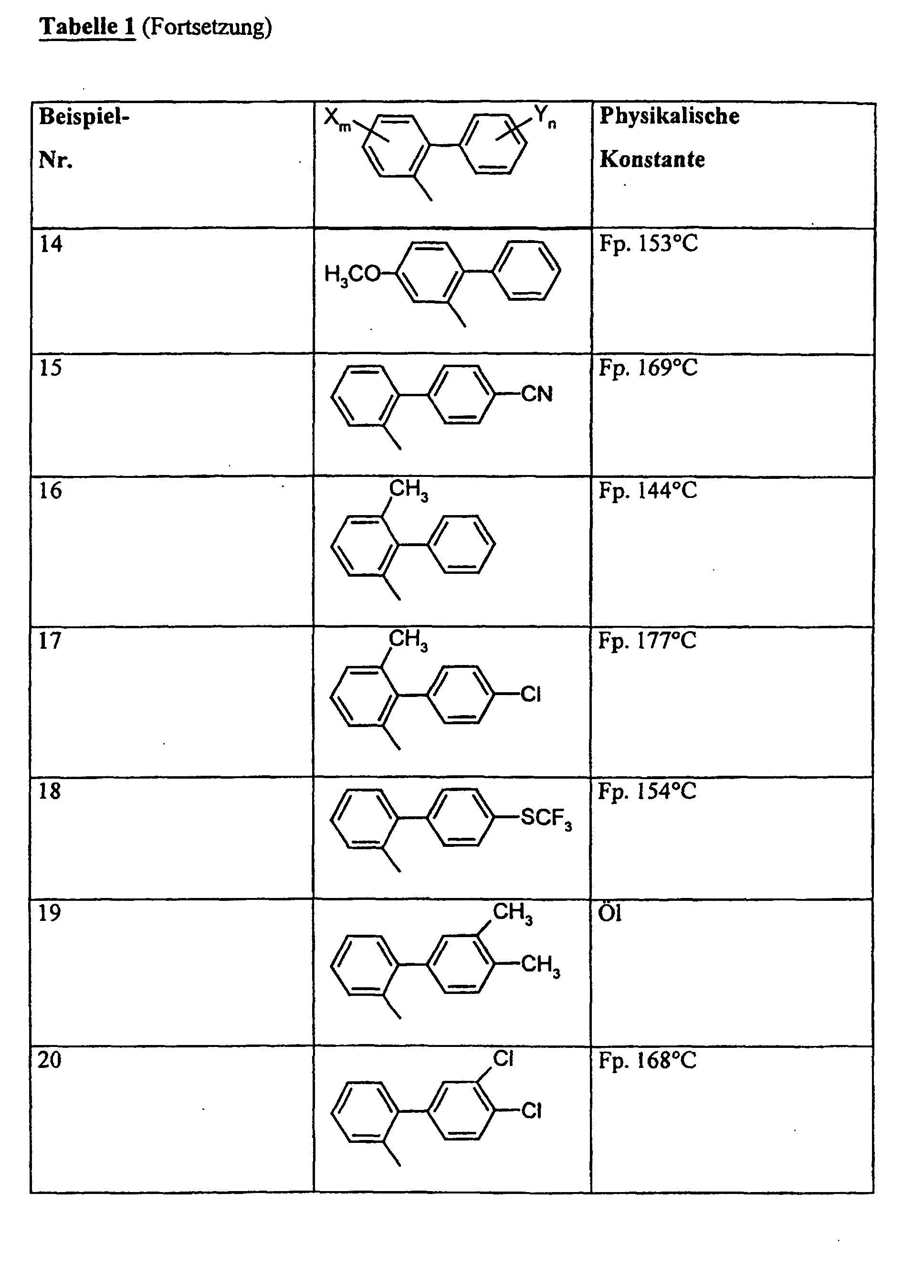

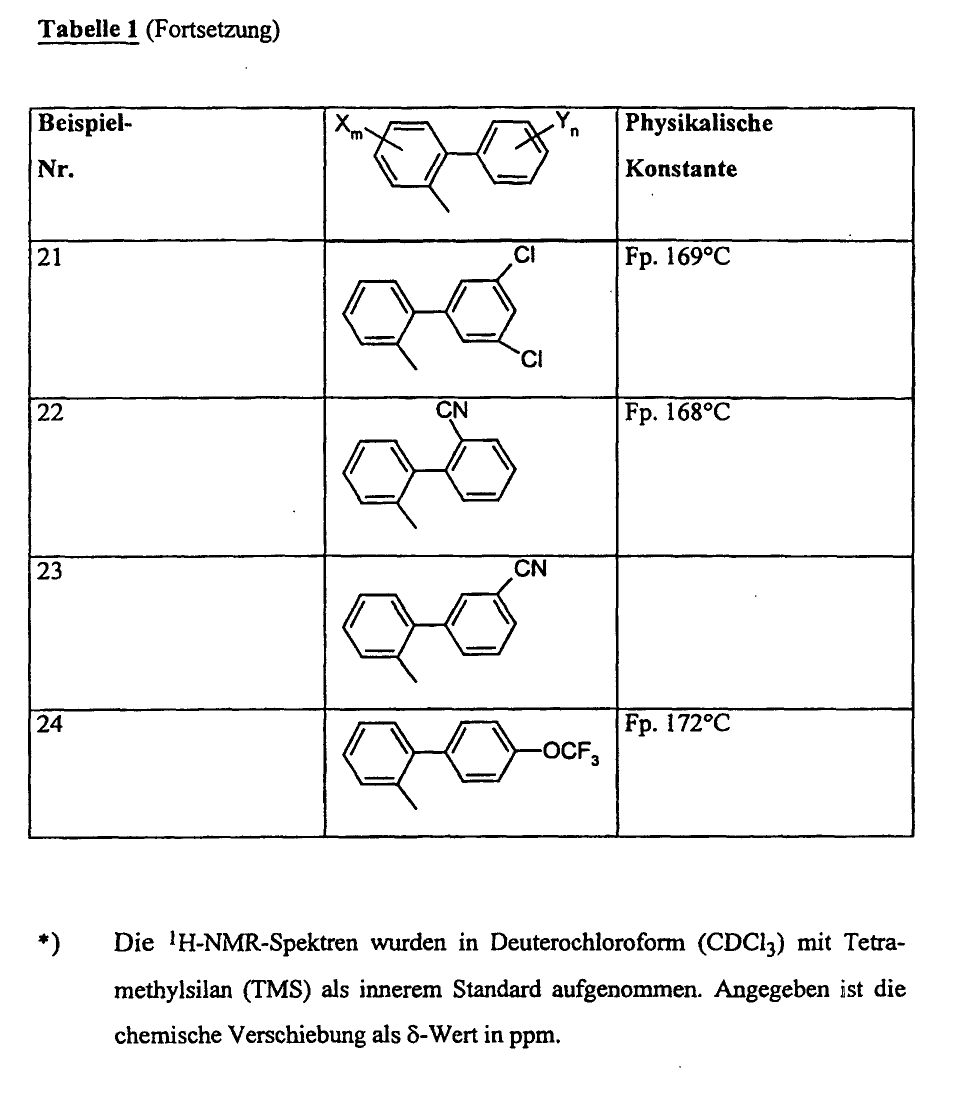

### Verwendungsbeispiele

### Beispiel A

### Podosphaera-Test (Apfel) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

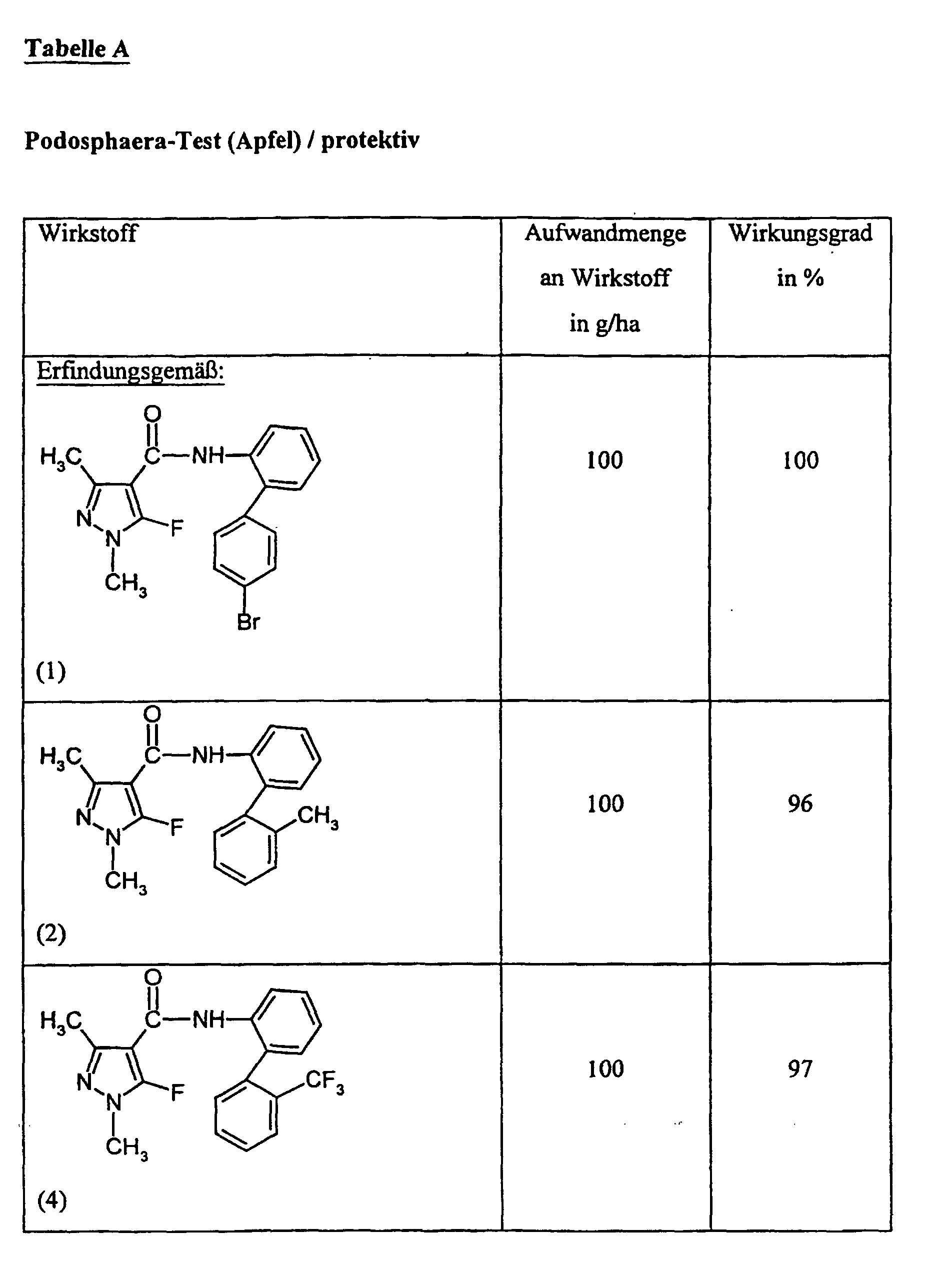

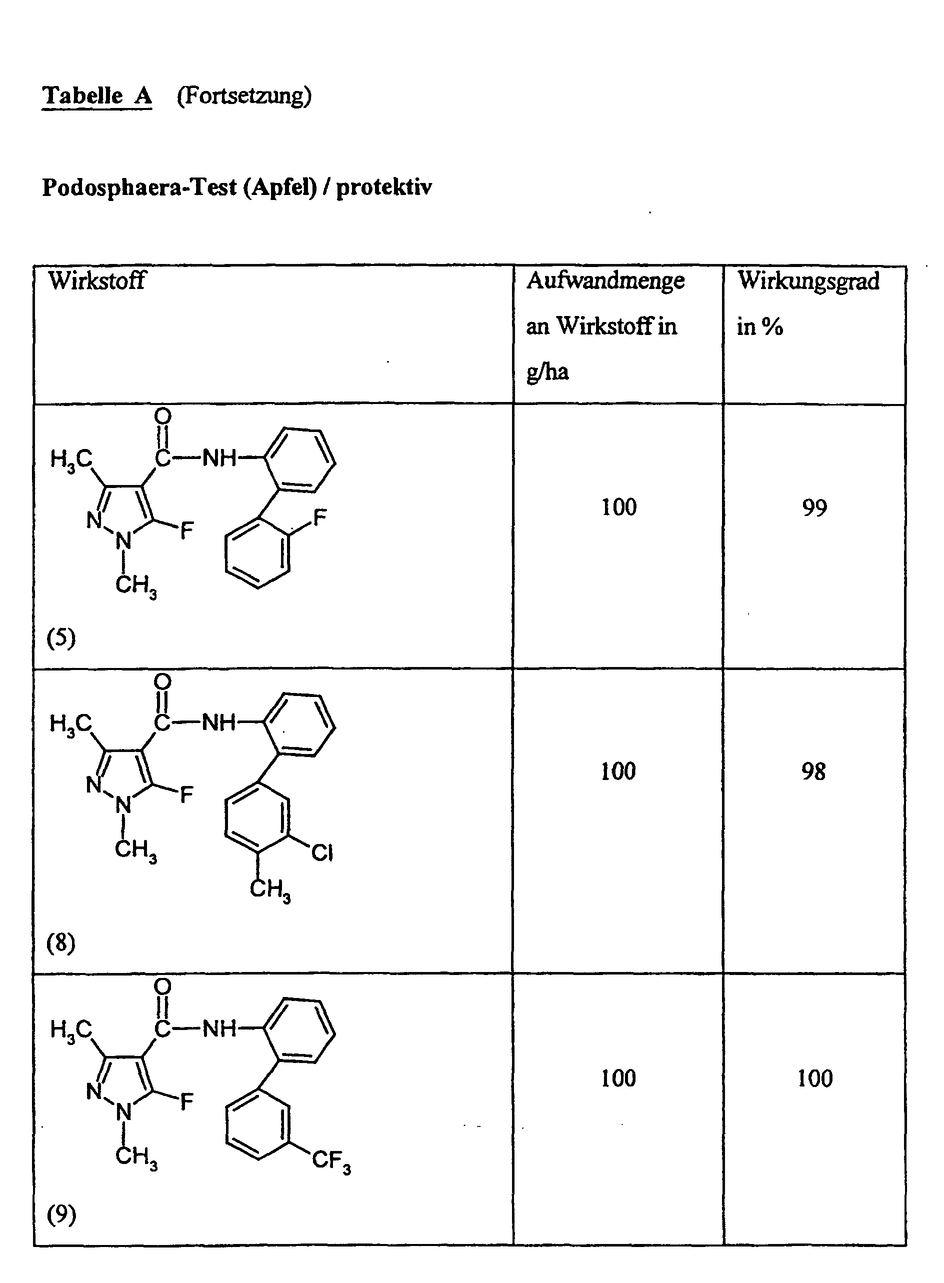

### Beispiel B

### Sphaerotheca-Test (Gurke) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von Sphaerotheca fuliginea inokuliert. Die Pflanzen werden dann bei ca. 23°C und einer relativen Luftfeuchtigkeit von ca. 70 % im Gewächshaus aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

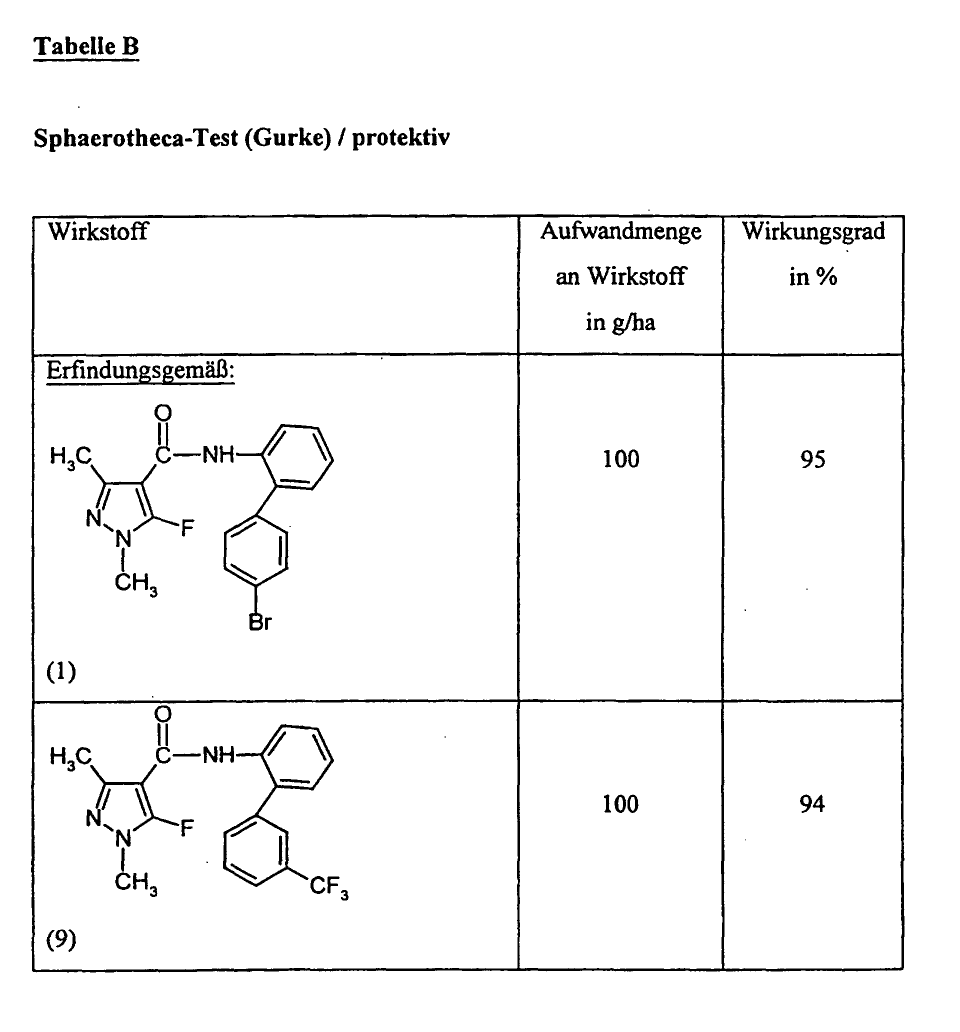

### Beispiel C

### Venturia-Test (Apfel) / protektiv

| | | |
|---|---|---|
| Lösungsmittel | 47 | Gewichtsteile Aceton |
| Emulgator | 3 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Konidiensuspension des Apfelschorferregers Venturia inaequalis inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

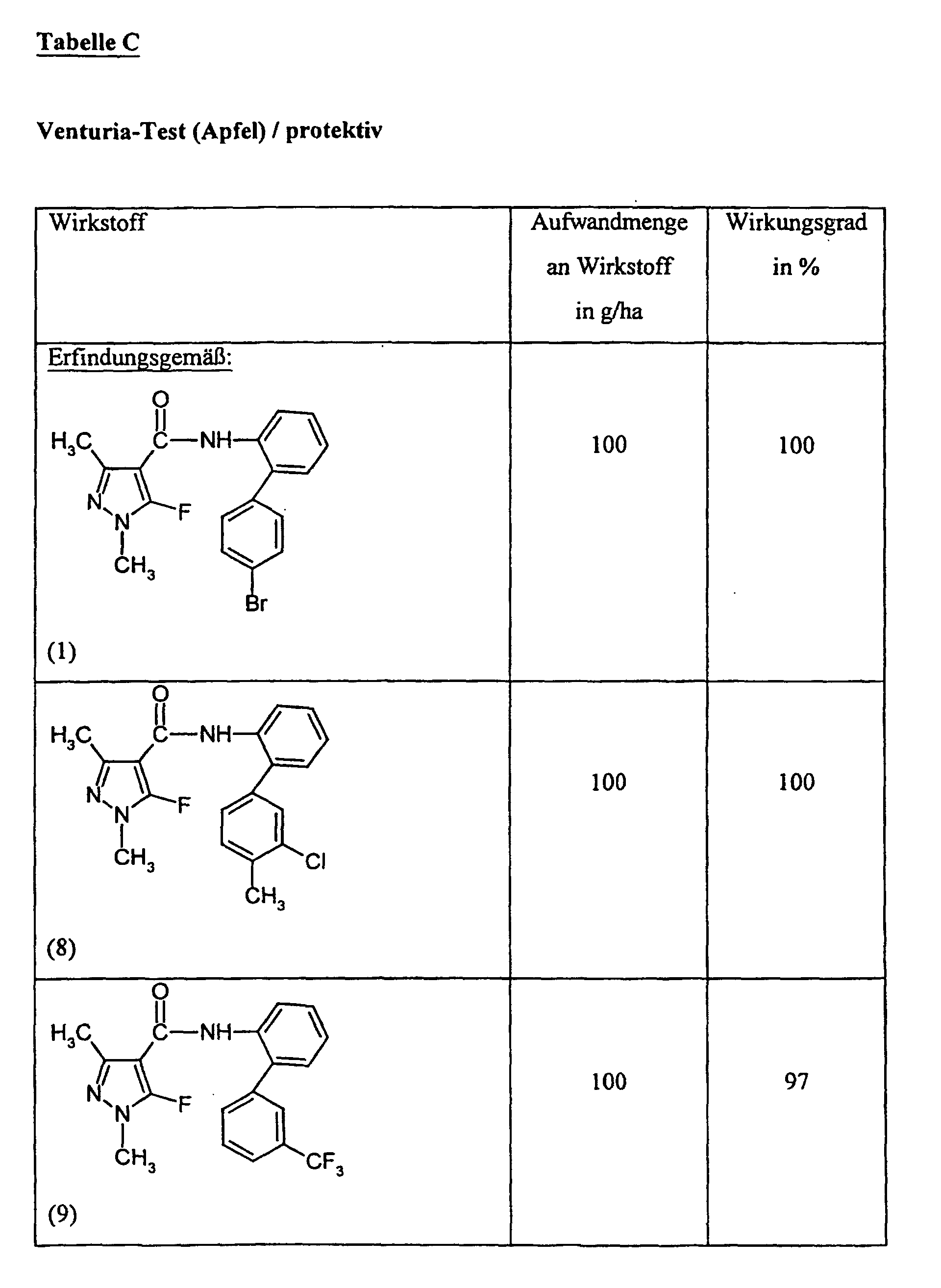

### Beispiel D

### Erysiphe-Test (Gerste) / protektiv

| | | |
|---|---|---|
| Lösungsmittel | 48,8 | Gewichtsteile N,N-Dimethylformamid |
| Emulgator | 1,2 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Getreidepflanzen mit der Wirkstoffzubereitung in der angegebenen Aufwandmenge. 1 Tag nach der Behandlung werden die Pflanzen mit Sporen von Erysiphe graminis f. sp. hordei inokuliert. Anschließend werden die Pflanzen in einem Gewächshaus bei 70 % relativer Luftfeuchtigkeit und einer Temperatur von 18 °C aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

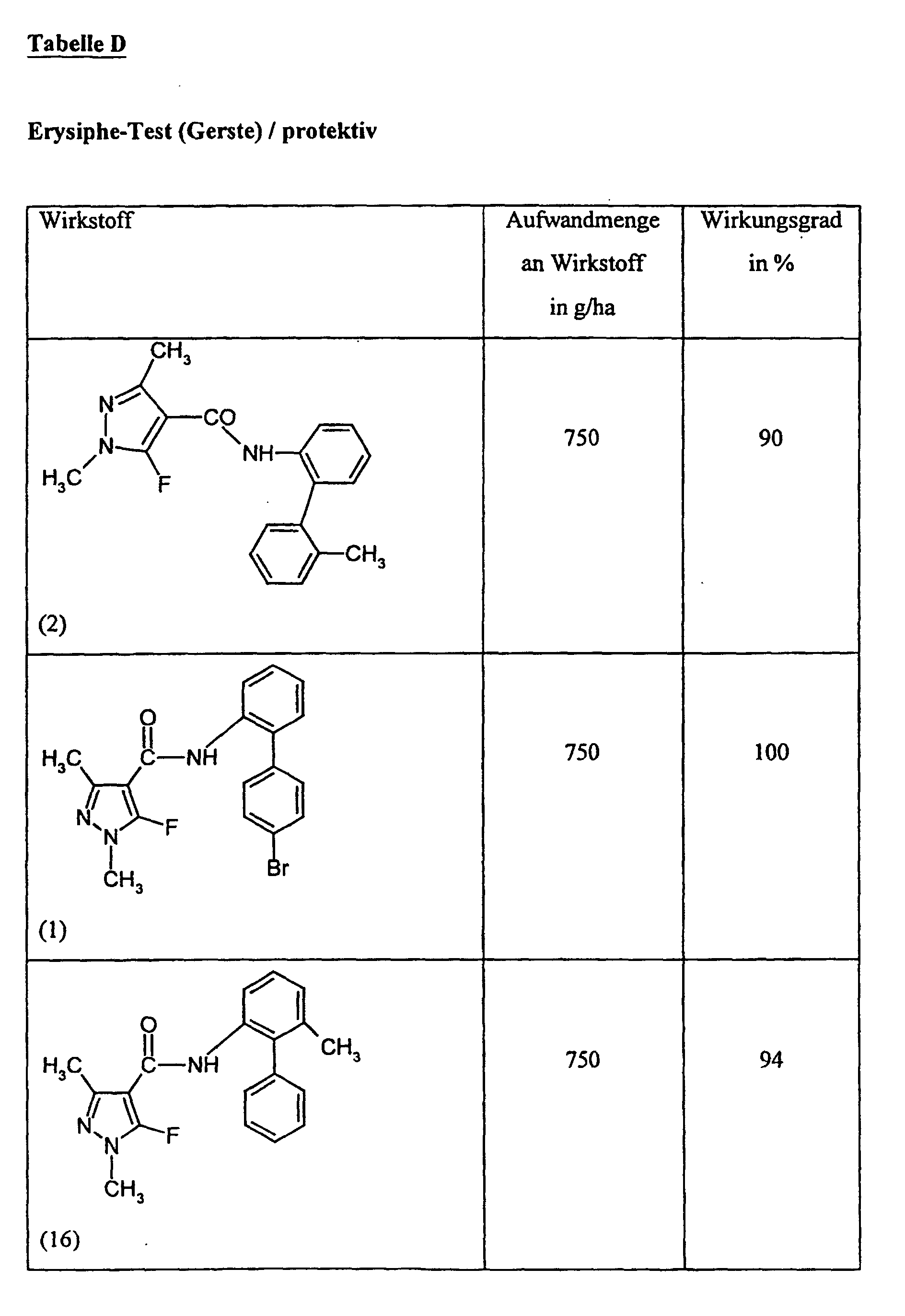

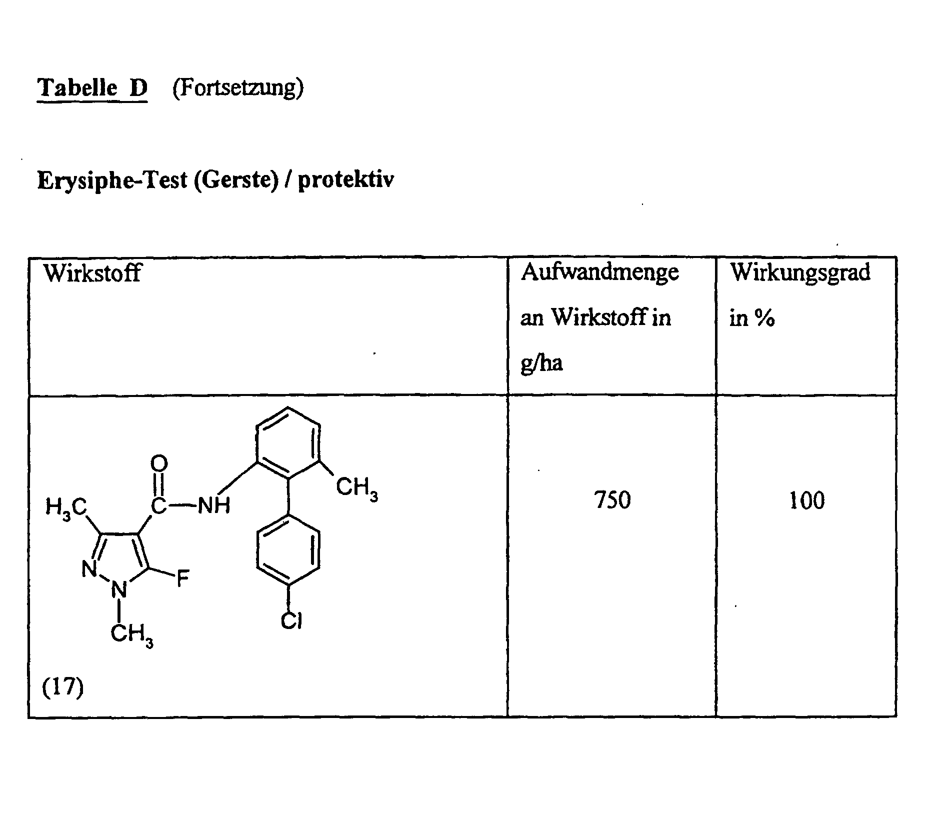

### Beispiel E

### Pyrenophora teres-Test (Gerste) / protektiv

| | | |
|---|---|---|
| Lösungsmittel | 25 | Gewichtsteile N,N-Dimethylacetamid |
| Emulgator | 0,6 | Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Pyrenophora teres besprüht. Die Pflanzen verbleiben 48 Stunden bei 20°C und 100 % relativer Luftfeuchtigkeit in einer Inkubationskabine.

Die Pflanzen werden dann in einem Gewächshaus bei einer Tempoeratur von ca. 20°C und relativen Luftfeuchtigkeit von ca. 80 % aufgestellt.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, daß kein Befall beobachtet wird.

Wirkstoffe, Aufwandmengen und Versuchsergebnisse gehen aus der folgenden Tabelle hervor.

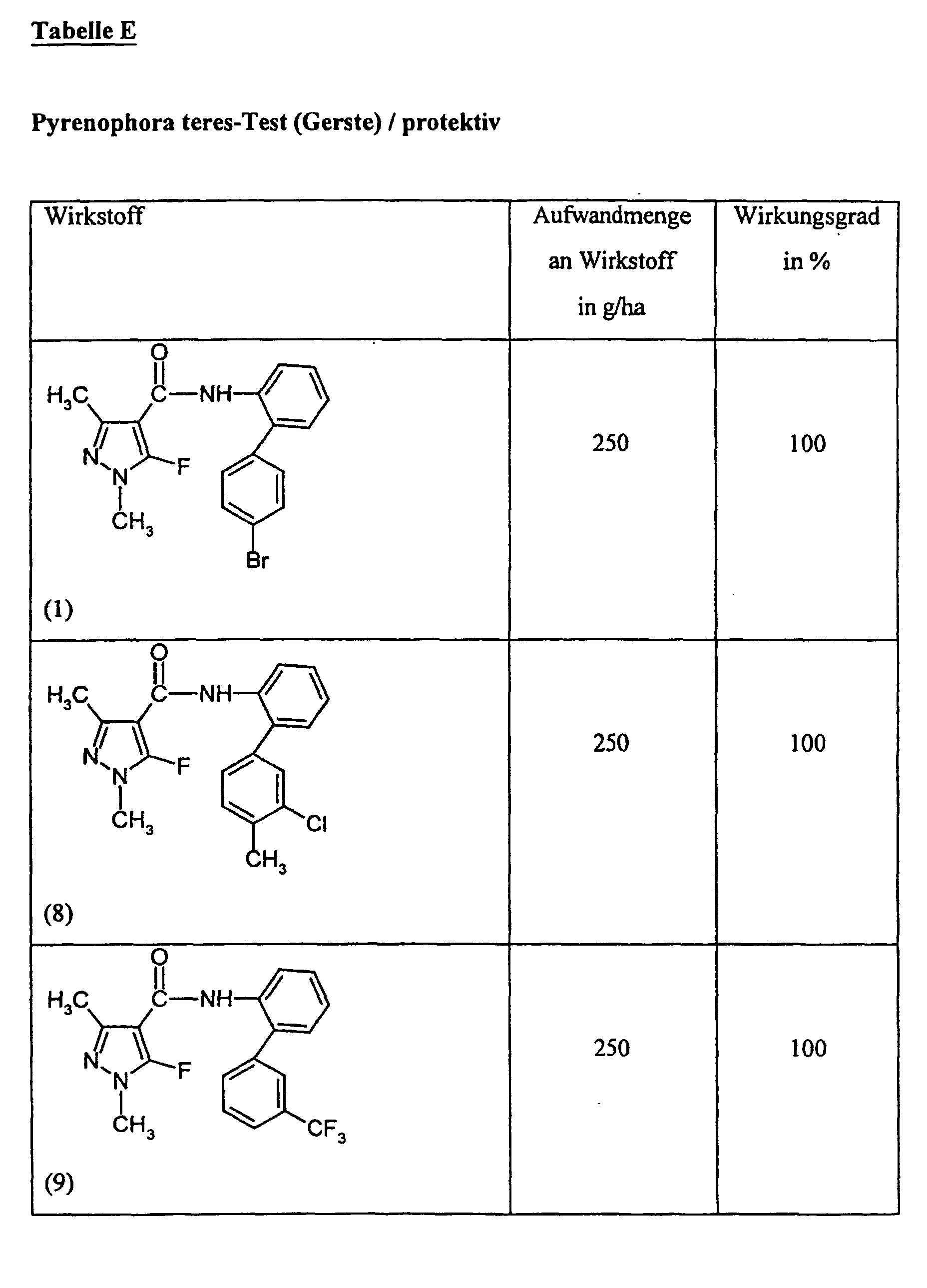

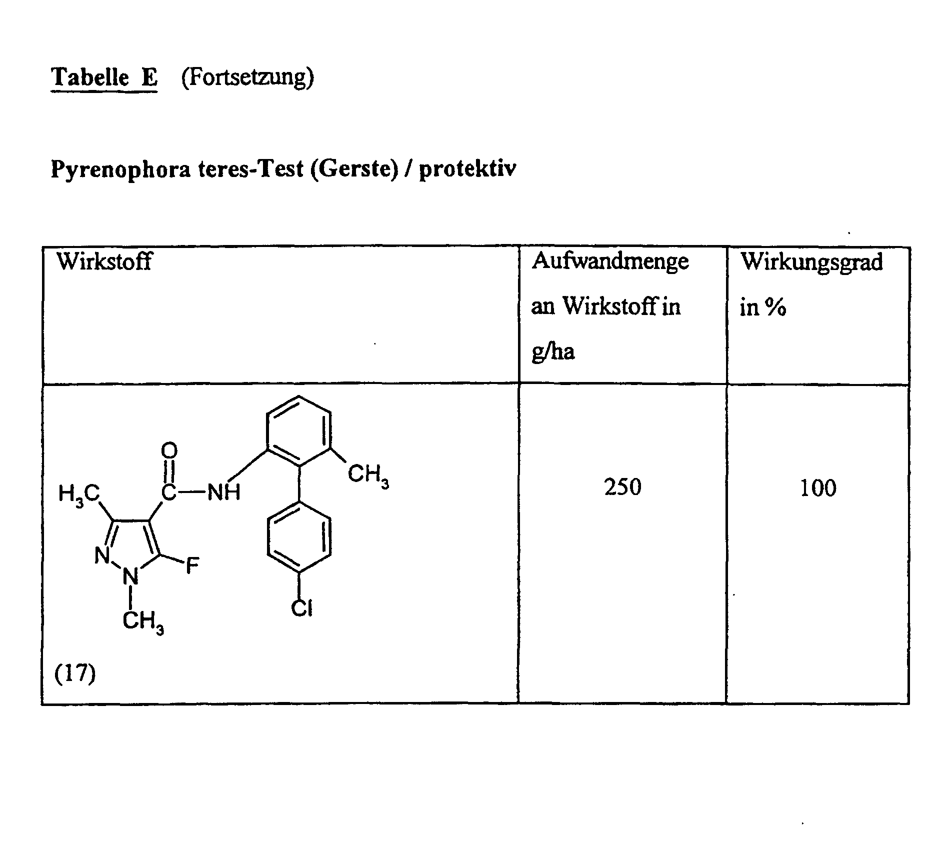

## Patentansprüche

1. Pyrazol-carboxanilide der Formel 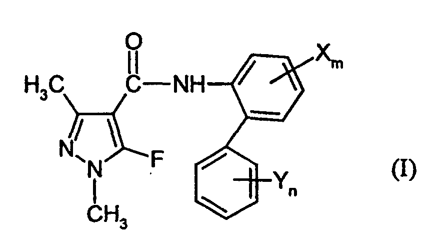 in welcher
a)
m für die Zahl 0 steht,
n für die Zahl 1 steht und
Y für 2-Chlor, 2-Fluor, 4-Brom, 2-Methyl, 2-Trifluormethyl, 3-Trifluormethyl,
für Nitro, Cyano, Hydroxy, Carboxyl, Halogenalkoxy mit 1 bis 6 Kohlenstoffatomen und 1 bis 5 Halogenatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
oder
b)
m für die Zahl 0 steht,
n für die Zahlen 2 oder 3 steht und
Y für Halogen, Nitro, Cyano, Hydroxy, Carboxyl, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
oder
c)
m für die Zahl 1 steht,
X für Chlor, Brom, Nitro, Cyano, Hydroxy, Carboxyl, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, HalogenalkoXy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht,
n für ganze Zahlen von 0 bis 3 steht und
Y für Halogen, Nitro, Cyano, Hydroxy, Carboxyl, Alkyl mit 1 bis 8 Kohlenstoffatomen, Halogenalkyl mit 1 bis 6 Kohlenstoffatomen und 1 bis 5 Halogenatomen, Alkoxy mit 1 bis 8 Kohlenstoffatomen, Halogenalkoxy mit 1 bis 6 Kohlenstoffatomen und 1 bis 5 Halogenatomen, Alkylthio mit 1 bis 8 Kohlenstoffatomen, Halogenalkylthio mit 1 bis 6 Kohlenstoffatomen und 1 bis 5 Halogenatomen, Alkenyloxy mit 2 bis 8 Kohlenstoffatomen, Alkinyloxy mit 2 bis 8 Kohlenstoffatomen, Cycloalkyl mit 3 bis 8 Kohlenstoffatomen, Carbalkoxy mit 1 bis 8 Kohlenstoffatomen im Alkoxyteil oder Alkoximinoalkyl mit 1 bis 6 Kohlenstoffatomen im Alkoxyteil und 1 bis 6 Kohlenstoffatomen im Alkylteil steht.

2. Pyrazol-carboxanilide der Formel (I) gemäß Anspruch 1, worin
a)
m für die Zahl 0 steht,
n für die Zahl 1 steht und
Y für 2-Chlor, 2-Fluor, 4-Brom, 2-Methyl, 2-Trifluormethyl, 3-Trifluormethyl,
für Nitro, Cyano, Hydroxy, Carboxyl, Halogenalkoxy mit 1 oder 2 Kohlenstoffatomen und 1 bis 5 Fluor-, Chlor- und/oder Bromatomen, Halogenalkylthio mit 1 oder 2 Kohlenstoffatomen und 1 bis 5 Fluor-, Chlor und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
oder
b)
m für die Zahl 0 steht,
n für die Zahlen 2 oder 3 steht und
Y für Fluor, Chlor, Brom, Nitro, Cyano, Hydroxy, Carboxyl, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor- und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
oder
c)
m für die Zahl 1 steht,
X für Chlor, Brom, Nitro, Cyano, Hydroxy, Carboxyl, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht,
n für ganze Zahlen von 0 bis 3 steht und
Y für Fluor, Chlor, Brom, Nitro, Cyano, Hydroxy, Carboxyl, Alkyl mit 1 bis 6 Kohlenstoffatomen, Halogenalkyl mit 1 oder 2 Kohlenstoffatomen und 1 bis 5 Fluor-, Chlor- und/oder Bromatomen, Alkoxy mit 1 bis 6 Kohlenstoffatomen, Halogenalkoxy mit 1 oder 2 Kohlenstoffatomen und 1 bis 5 Fluor-, Chlor- und/oder Bromatomen, Alkylthio mit 1 bis 6 Kohlenstoffatomen, Halogenalkylthio mit 1 oder 2 Kohlenstoffatomen und 1 bis 5 Fluor-, Chlor- und/oder Bromatomen, Alkenyloxy mit 2 bis 6 Kohlenstoffatomen, Alkinyloxy mit 2 bis 6 Kohlenstoffatomen, Cycloalkyl mit 3 bis 7 Kohlenstoffatomen, Carbalkoxy mit 1 bis 4 Kohlenstoffatomen im Alkoxyteil oder für Alkoximinoalkyl mit 1 bis 4 Kohlenstoffatomen im Alkoxyteil und 1 bis 4 Kohlenstoffatomen im Alkylteil steht.

3. Pyrazol-carboxanilid gemäß Anspruch 1, **gekennzeichnet durch** die Formel

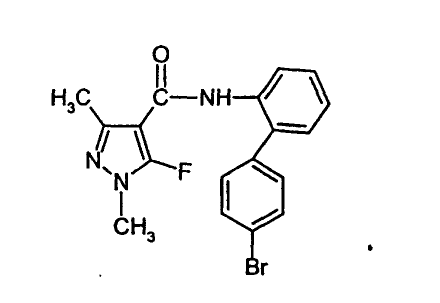

4. Pyrazol-carboxanilid gemäß Anspruch 1, **gekennzeichnet durch** die Formel

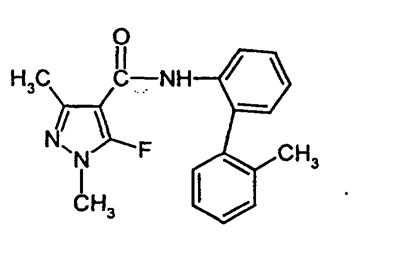

5. Pyrazol-carboxanilid gemäß Anspruch 1, **gekennzeichnet durch** die Formel

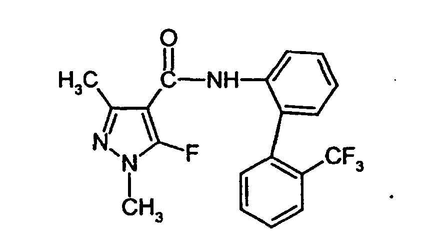

6. Verfahren zur Herstellung von Pyrazol-carboxaniliden der Formel (I) gemäß Anspruch 1, **dadurch gekennzeichnet, daß** man Säurehalogenide der Formel 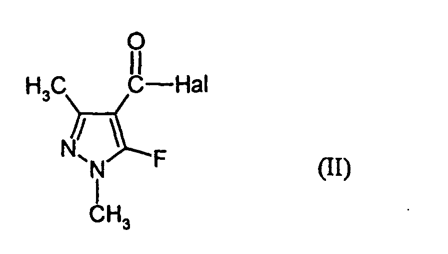 in welcher
Hal für Halogen steht,
mit Anilin-Derivaten der Formel 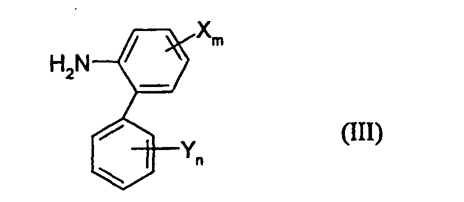 in welcher
X, Y, m und n die oben angegebenen Bedeutungen haben,
gegebenenfalls in Gegenwart eines Säurebindemittels und gegebenenfalls in Gegenwart eines Verdünnungsmittels umsetzt.

7. Mittel zur Bekämpfung unerwünschter Mikroorganismen, **gekennzeichnet durch** einen Gehalt an mindestens einem Pyrazol-caboxanilid der Formel (I) gemäß Anspruch 1 neben Streckmitteln und/oder oberflächenaktiven Stoffen.

8. Verwendung von Pyrazol-carboxaniliden der Formel (I) gemäß Anspruch 1 zur Bekämpfung unerwünschter Mikroorganismen mit Ausnahme von therapeutischen oder diagnostischen Verwendungen am oder im menschlichen oder tierischichen Körper.

9. Verfahren zur Bekämpfung unerwünschter Mikroorganismen, **dadurch gekennzeichnet, daß** man Pyrazol-carboxanilide der Formel (I) gemäß Anspruch 1 auf die Mikroorganismen und/oder deren Lebensraum ausbringt mit Ausnahme von therapeutischen oder diagnostischen Verfahren am oder im menschlichen oder tierischen Körper.

10. Verfahren zur Herstellung von Mitteln zur Bekämpfung unerwünschter Mikroorganismen, **dadurch gekennzeichnet, daß** man Pyrazol-carboxanilide der Formel (I) gemäß Anspruch 1 mit Streckmitteln und/oder oberflächenaktiven Stoffen vermischt.

## Claims

1. Pyrazole-carboxanilides of the formula 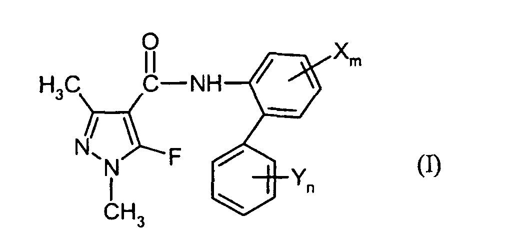 in which
a)
m represents the number 0,
n represents the number 1 and
Y represents 2-chloro, 2-fluoro, 4-bromo, 2-methyl, 2-trifluoromethyl, 3-trifluoromethyl,
represents nitro, cyano, hydroxyl, carboxyl, halogenoalkoxy having 1 to 6 carbon atoms and 1 to 5 halogen atoms, halogenoalkylthio having 1 to 6 carbon atoms and 1 to 5 halogen atoms, alkenyloxy having 2 to 8 carbon atoms, alkinyloxy having 2 to 8 carbon atoms, cycloalkyl having 3 to 8 carbon atoms, carbalkoxy having 1 to 8 carbon atoms in the alkoxy moiety or alkoximinoalkyl having 1 to 6 carbon atoms in the alkoxy moiety and 1 to 6 carbon atoms in the alkyl moiety,
or
b)
m represents the number 0,
n represents the number 2 or 3 and
Y represents halogen, nitro, cyano, hydroxyl, carboxyl, alkyl having 1 to 8 carbon atoms, halogenoalkyl having 1 to 6 carbon atoms and 1 to 5 halogen atoms, alkoxy having 1 to 8 carbon atoms, halogenoalkoxy having 1 to 6 carbon atoms and 1 to 5 halogen atoms, alkylthio having 1 to 8 carbon atoms, halogenoalkylthio having 1 to 6 carbon atoms and 1 to 5 halogen atoms, alkenyloxy having 2 to 8 carbon atoms, alkinyloxy having 2 to 8 carbon atoms, cycloalkyl having 3 to 8 carbon atoms, carbalkoxy having 1 to 8 carbon atoms in the alkoxy moiety or alkoximinoalkyl having 1 to 6 carbon atoms in the alkoxy moiety and 1 to 6 carbon atoms in the alkyl moiety,
or
c)
m represents the number 1,
X represents chlorine, bromine, nitro, cyano, hydroxyl, carboxyl, alkyl having 1 to 8 carbon atoms, halogenoalkyl having 1 to 6 carbon atoms and 1 to 5 halogen atoms, alkoxy having 1 to 8 carbon atoms, halogenoalkoxy having 1 to 6 carbon atoms and 1 to 5 halogen atoms, alkylthio having 1 to 8 carbon atoms, halogenoalkylthio having 1 to 6 carbon atoms and 1 to 5 halogen atoms, alkenyloxy having 2 to 8 carbon atoms, alkinyloxy having 2 to 8 carbon atoms, cycloalkyl having 3 to 8 carbon atoms, carbalkoxy having 1 to 8 carbon atoms in the alkoxy moiety or alkoximinoalkyl having 1 to 6 carbon atoms in the alkoxy moiety and 1 to 6 carbon atoms in the alkyl moiety,
n represents integers from 0 to 3 and
Y represents halogen, nitro, cyano, hydroxyl, carboxyl, alkyl having 1 to 8 carbon atoms, halogenoalkyl having 1 to 6 carbon atoms and 1 to 5 halogen atoms, alkoxy having 1 to 8 carbon atoms, halogenoalkoxy having 1 to 6 carbon atoms and 1 to 5 halogen atoms, alkylthio having 1 to 8 carbon atoms, halogenoalkylthio having 1 to 6 carbon atoms and 1 to 5 halogen atoms, alkenyloxy having 2 to 8 carbon atoms, alkinyloxy having 2 to 8 carbon atoms, cycloalkyl having 3 to 8 carbon atoms, carbalkoxy having 1 to 8 carbon atoms in the alkoxy moiety or alkoximinoalkyl having 1 to 6 carbon atoms in the alkoxy moiety and 1 to 6 carbon atoms in the alkyl moiety.

2. Pyrazole-carboxanilides of the formula (I) according to claim 1, in which
a)
m represents the number 0,
n represents the number 1 and
Y represents 2-chloro, 2-fluoro, 4-bromo, 2-methyl, 2-trifluoromethyl, 3-trifluoromethyl, represents nitro, cyano, hydroxyl, carboxyl, halogenoalkoxy having 1 or 2 carbon atoms and 1 to 5 fluorine, chlorine and/or bromine atoms, halogenoalkylthio having 1 or 2 carbon atoms and 1 to 5 fluorine, chlorine and/or bromine atoms, alkenyloxy having 2 to 6 carbon atoms, alkinyloxy having 2 to 6 carbon atoms, cycloalkyl having 3 to 7 carbon atoms, carbalkoxy having 1 to 4 carbon atoms in the alkoxy moiety or represents alkoximinoalkyl having 1 to 4 carbon atoms in the alkoxy moiety and 1 to 4 carbon atoms in the alkyl moiety,
or
b)
m represents the number 0,
n represents the number 2 or 3 and
Y represents fluorine, chlorine, bromine, nitro, cyano, hydroxyl, carboxyl, alkyl having 1 to 6 carbon atoms, halogenoalkyl having 1 or 2 carbon atoms and 1 to 5 fluorine, chlorine and/or bromine atoms, alkoxy having 1 to 6 carbon atoms, halogenoalkoxy having 1 or 2 carbon atoms and 1 to 5 fluorine, chlorine and/or bromine atoms, alkylthio having 1 to 6 carbon atoms, halogenoalkylthio having 1 or 2 carbon atoms and 1 to 5 fluorine, chlorine and/or bromine atoms, alkenyloxy having 2 to 6 carbon atoms, alkinyloxy having 2 to 6 carbon atoms, cycloalkyl having 3 to 7 carbon atoms, carbalkoxy having 1 to 4 carbon atoms in the alkoxy moiety or represents alkoximinoalkyl having 1 to 4 carbon atoms in the alkoxy moiety and 1 to 4 carbon atoms in the alkyl moiety,
or
c)
m represents the number 1,
X represents chlorine, bromine, nitro, cyano, hydroxyl, carboxyl, alkyl having 1 to 6 carbon atoms, halogenoalkyl having 1 or 2 carbon atoms and 1 to 5 fluorine, chlorine and/or bromine atoms, alkoxy having 1 to 6 carbon atoms, halogenoalkoxy having 1 or 2 carbon atoms and 1 to 5 fluorine, chlorine and/or bromine atoms, alkylthio having 1 to 6 carbon atoms, halogenoalkylthio having 1 or 2 carbon atoms or 1 to 5 fluorine, chlorine, and/or bromine atoms, alkenyloxy having 2 to 6 carbon atoms, alkinyloxy having 2 to 6 carbon atoms, cycloalkyl having 3 to 7 carbon atoms, carbalkoxy having 1 to 4 carbon atoms in the alkoxy moiety or represents alkoximinoalkyl having 1 to 4 carbon atoms in the alkoxy moiety and 1 to 4 carbon atoms in the alkyl moiety,
n represents integers from 0 to 3 and
Y represents fluorine, chlorine, bromine, nitro, cyano, hydroxyl, carboxyl, alkyl having 1 to 6 carbon atoms, halogenoalkyl having 1 or 2 carbon atoms and 1 to 5 fluorine, chlorine and/or bromine atoms, alkoxy having 1 to 6 carbon atoms, halogenoalkoxy having 1 or 2 carbon atoms and 1 to 5 fluorine, chlorine and/or bromine atoms, alkylthio having 1 to 6 carbon atoms, halogenoalkylthio having 1 or 2 carbon atoms and 1 to 5 fluorine, chlorine and/or bromine atoms, alkenyloxy having 2 to 6 carbon atoms, alkinyloxy having 2 to 6 carbon atoms, cycloalkyl having 3 to 7 carbon atoms, carboalkoxy having 1 to 4 carbon atoms in the alkoxy moiety or represents alkoximinoalkyl having 1 to 4 carbon atoms in the alkoxy moiety and 1 to 4 carbon atoms in the alkyl moiety.

3. Pyrazole-carboxanilide according to Claim 1, **characterized by** the formula

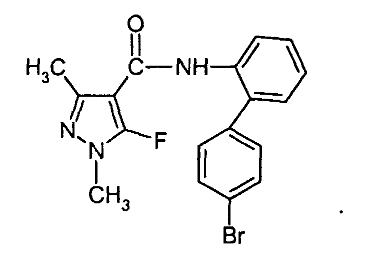

4. Pyrazole-carboxanilide according to Claim 1, **characterized by** the formula

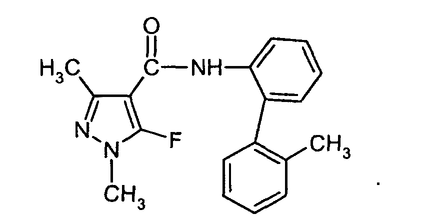

5. Pyrazole-carboxanilide according to Claim 1, **characterized by** the formula

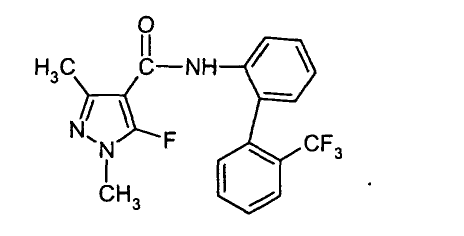

6. Process for preparing pyrazole-carboxanilides of the formula (I) according to Claim 1, **characterized in that** acyl halides of the formula 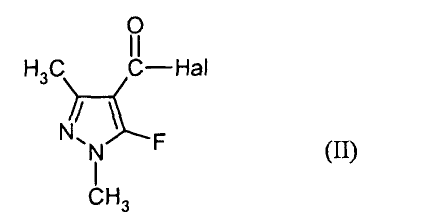 in which
Hal represents halogen,
are reacted with aniline derivatives of the formula 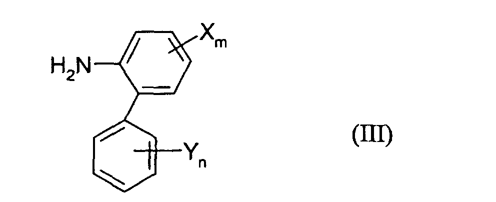 in which
X, Y, m and n are each as defined above,
if appropriate in the presence of an acid binder and if appropriate in the presence of a diluent.

7. Compositions for controlling undesirable microorganisms, **characterized in that** they comprise at least one pyrazole-carboxanilide of the formula (I) according to Claim 1, in addition to extenders and/or surfactants.

8. Use of pyrazole-carboxanilides of the formula (I) according to Claim 1 for controlling undesirable microorganisms with the exception of therapeutic or diagnostic uses on or in the human or animal body.

9. Method for controlling undesirable microorganisms, **characterized in that** pyrazole-carboxanilides of the formula (I) according to Claim 1 are applied to the microorganisms and/or their habitat with the exception of therapeutic or diagnostic methods on or in the human or animal body.

10. Process for preparing compositions for controlling undesirable microorganisms, **characterized in that** pyrazole-carboxanilides of the formula (I) according to claim 1 are mixed with extenders and/or surfactants.

## Revendications

1. Pyrazole-carboxanilides de formule 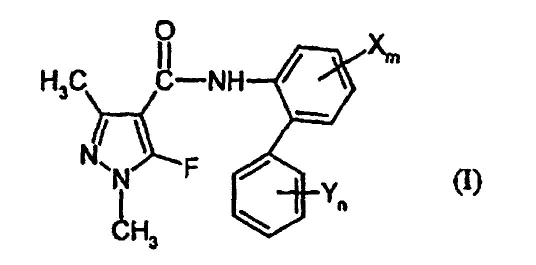 dans laquelle
a)
m est le nombre 0,
n est le nombre 1 et
Y est un reste 2-chloro, 2-fluoro, 4-bromo, 2-méthyle, 2-trifluorométhyle, 3-trifluorométhyle, un reste nitro, cyano, hydroxy, carboxyle, halogénalkoxy ayant 1 à 6 atomes de carbone et 1 à 5 atomes d'halogène, halogénalkylthio ayant 1 à 6 atomes de carbone et 1 à 5 atomes d'halogène, alcényloxy ayant 2 à 8 atomes de carbone, alcynyloxy ayant 2 à 8 atomes de carbone, cycloalkyle ayant 3 à 8 atomes de carbone, carbalkoxy ayant 1 à 8 atomes de carbone dans la partie alkoxy, ou alkoximinoalkyle ayant 1 à 6 atomes de carbone dans la partie alkoxy et 1 à 6 atomes de carbone dans la partie alkyle,
ou bien
b)
m est le nombre 0,
n représente les nombres 2 ou 3 et
Y est un halogène, un groupe nitro, cyano, hydroxy, carboxyle, un reste alkyle ayant 1 à 8 atomes de carbone, halogénalkyle ayant 1 à 6 atomes de carbone et 1 à 5 atomes d'halogène, alkoxy ayant 1 à 8 atomes de carbone, halogénalkoxy ayant 1 à 6 atomes de carbone et 1 à 5 atomes d'halogène, alkylthio ayant 1 à 8 atomes de carbone, halogénalkylthio ayant 1 à 6 atomes de carbone et 1 à 5 atomes d'halogène, alcényloxy ayant 2 à 8 atomes de carbone, alcynyloxy ayant 2 à 8 atomes de carbone, cycloalkyle ayant 3 à 8 atomes de carbone, carbalkoxy ayant 1 à 8 atomes de carbone dans la partie alkoxy ou alkoximinoalkyle ayant 1 à 6 atomes de carbone dans la partie alkoxy et 1 à 6 atomes de carbone dans la partie alkyle,
ou bien
c)
m est le nombre 1,
X représente le chlore, le brome, un groupe nitro, cyano, hydroxy, carboxyle, un reste alkyle ayant 1 à 8 atomes de carbone, halogénalkyle ayant 1 à 6 atomes de carbone et 1 à 5 atomes d'halogène, alkoxy ayant 1 à 8 atomes de carbone, halogénalkoxy ayant 1 à 6 atomes de carbone et 1 à 5 atomes d'halogène, alkylthio ayant 1 à 8 atomes de carbone, halogénalkylthio ayant 1 à 6 atomes de carbone et 1 à 5 atomes d'halogène, alcényloxy ayant 2 à 8 atomes de carbone, alcynyloxy ayant 2 à 8 atomes de carbone, cycloalkyle ayant 3 à 8 atomes de carbone, carbalkoxy ayant 1 à 8 atomes de carbone dans la partie alkoxy ou alkoximinoalkyle ayant 1 à 6 atomes de carbone dans la partie alkoxy et 1 à 6 atomes de carbone dans la partie alkyle,
n représente des nombres entiers de 0 à 3 et
Y est un halogène, un groupe nitro, cyano, hydroxy, carboxyle, un reste alkyle ayant 1 à 8 atomes de carbone, halogénalkyle ayant 1 à 6 atomes de carbone et 1 à 5 atomes d'halogène, alkoxy ayant 1 à 8 atomes de carbone, halogénalkoxy ayant 1 à 6 atomes de carbone et 1 à 5 atomes d'halogène, alkylthio ayant 1 à 8 atomes de carbone, halogénalkylthio ayant 1 à 6 atomes de carbone et 1 à 5 atomes d'halogène, alcényloxy ayant 2 à 8 atomes de carbone, alcynyloxy ayant 2 à 8 atomes de carbone, cycloalkyle ayant 3 à 8 atomes de carbone, carbalkoxy ayant 1 à 8 atomes de carbone dans la partie alkoxy ou alkoximinoalkyle ayant 1 à 6 atomes de carbone dans la partie alkoxy et 1 à 6 atomes de carbone dans la partie alkyle,

2. Pyrazole-carboxanilides de formule (I) suivant la revendication 1, formule dans laquelle
a)
m est le nombre 0,
n est le nombre 1 et
Y est un reste 2-chloro, 2-fluoro, 4-bromo, 2-méthyle, 2-trifluorométhyle, 3-trifluorométhyle, un reste nitro, cyano, hydroxy, carboxyle, halogénalkoxy ayant 1 ou 2 atomes de carbone et 1 à 5 atomes de fluor, de chlore et/ou de brome, halogénalkylthio ayant 1 ou 2 atomes de carbone et 1 à 5 atomes de fluor, de chlore et/ou de brome, alcényloxy ayant 2 à 6 atomes de carbone, alcynyloxy ayant 2 à 6 atomes de carbone, cycloalkyle ayant 3 à 7 atomes de carbone, carbalkoxy ayant 1 à 4 atomes de carbone dans la partie alkyle ou alkoximinoalkyle ayant 1 à 4 atomes de carbone dans la partie alkoxy et 1 à 4 atomes de carbone dans la partie alkyle,
ou bien
b)
m est le nombre 0,
n représente les nombres 2 ou 3 et
Y est le fluor, le chlore, le brome, un groupe nitro, cyano, hydroxy, carboxyle, un reste alkyle ayant 1 à 6 atomes de carbone, halogénalkyle ayant 1 ou 2 atomes de carbone et 1 à 5 atomes de fluor, de chlore et/ou de brome, alkoxy ayant 1 à 6 atomes de carbone, halogénalkoxy ayant 1 ou 2 atomes de carbone et 1 à 5 atomes de fluor, de chlore et/ou de brome, alkylthio ayant 1 à 6 atomes de carbone, halogénalkylthio ayant 1 ou 2 atomes de carbone et 1 à 5 atomes de fluor, de chlore et/ou de brome, alcényloxy ayant 2 à 6 atomes de carbone, alcynyloxy ayant 2 à 6 atomes de carbone, cycloalkyle ayant 3 à 7 atomes de carbone, carbalkoxy ayant 1 à 4 atomes de carbone dans la partie alkoxy ou un reste alkoximinoalkyle ayant 1 à 4 atomes de carbone dans la partie alkoxy et 1 à 4 atomes de carbone dans la partie alkyle,
ou bien
c)
m est le nombre 1,
X représente le chlore, le brome, un groupe nitro, cyano, hydroxy, carboxyle, un reste alkyle ayant 1 à 6 atomes de carbone, halogénalkyle ayant 1 ou 2 atomes de carbone et 1 à 5 atomes de fluor, de chlore et/ou de brome, alkoxy ayant 1 à 6 atomes de carbone, halogénalkoxy ayant 1 ou 2 atomes de carbone et 1 à 5 atomes de fluor, de chlore et/ou de brome, alkylthio ayant 1 à 6 atomes de carbone, halogénalkylthio ayant 1 ou 2 atomes de carbone et 1 à 5 atomes de fluor, de chlore et/ou de brome, alcényloxy ayant 2 à 6 atomes de carbone, alcynyloxy ayant 2 à 6 atomes de carbone, cycloalkyle ayant 3 à 7 atomes de carbone, carbalkoxy ayant 1 à 4 atomes de carbone dans la partie alkoxy ou un reste alkoximinoalkyle ayant 1 à 4 atomes de carbone dans la partie alkoxy et 1 à 4 atomes de carbone dans la partie alkyle,
n des nombres entiers de 0 à 3 et
Y représente le fluor, le chlore, le brome, un groupe nitro, cyano, hydroxy, carboxyle, un reste alkyle ayant 1 à 6 atomes de carbone, halogénalkyle ayant 1 ou 2 atomes de carbone et 1 à 5 atomes de fluor, de chlore et/ou de brome, alkoxy ayant 1 à 6 atomes de carbone, halogénalkoxy ayant 1 ou 2 atomes de carbone et 1 à 5 atomes de fluor, de chlore et/ou de brome, alkylthio ayant 1 à 6 atomes de carbone, halogénalkylthio ayant 1 ou 2 atomes de carbone et 1 à 5 atomes de fluor, de chlore et/ou de brome, alcényloxy ayant 2 à 6 atomes de carbone, alcynyloxy ayant 2 à 6 atomes de carbone, cycloalkyle ayant 3 à 7 atomes de carbone, carbalkoxy ayant 1 à 4 atomes de carbone dans la partie alkoxy ou un reste alkoximinoalkyle ayant 1 à 4 atomes de carbone dans la partie alkoxy et 1 à 4 atomes de carbone dans la partie alkyle,

3. Pyrazole-carboxanilide suivant la revendication 1, **caractérisé par** la formule

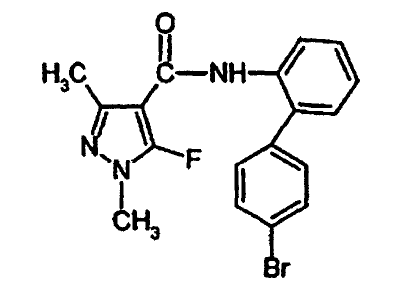

4. Pyrazole-carboxanilide suivant la revendication 1, **caractérisé par** la formule

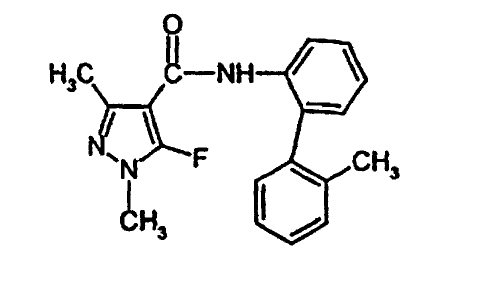

5. Pyrazole-carboxanilide suivant la revendication 1, **caractérisé par** la formule

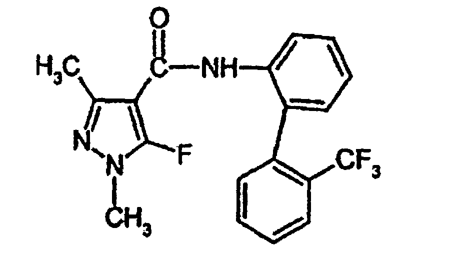

6. Procédé de production de pyrazole-carboxanilides de formule (I) suivant la revendication 1, **caractérisé en ce qu'**on fait réagir des halogénures d'acide de formule 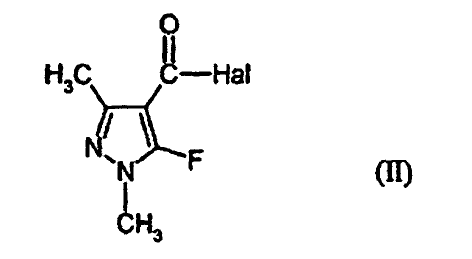 dans laquelle
Hal représente un halogène,
avec des dérivés d'aniline de formule 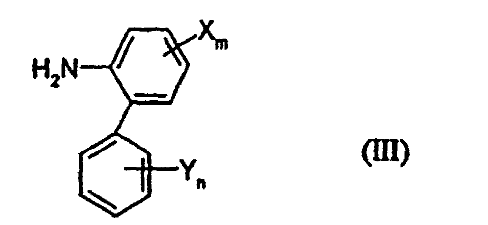 dans laquelle
X, Y, m et n ont les définitions indiquées ci-dessus, éventuellement en présence d'un accepteur d'acide et, le cas échéant, en présence d'un diluant.

7. Compositions destinées à combattre des microorganismes indésirables, **caractérisées par** une teneur en au moins un pyrazole-carboxanilide de formule (I) suivant la revendication 1, à côté de diluants et/ou d'agents tensioactifs.

8. Utilisation de pyrazole-carboxanilides de formule (I) suivant la revendication 1, pour combattre des microorganismes indésirables, à l'exception d'utilisations thérapeutiques ou diagnostiques sur ou dans le corps humain ou animal.

9. Procédé pour combattre des microorganismes indésirables, **caractérisé en ce qu'**on épand des pyrazoles-carboxanilides de formule (I) suivant la revendication 1 sur les microorganismes et/ou sur leur milieu, à l'exception de procédés thérapeutiques ou diagnostiques sur ou dans le corps humain ou animal.

10. Procédé de préparation de compositions destinées à combattre des microorganismes indésirables, **caractérisé en ce qu'**on mélange des pyrazole-carboxanilides de formule (I) suivant la revendication 1 avec des diluants et/ou des agents tensioactifs.
